# EUROPEAN PATENT SPECIFICATION

(11) **EP 1 407 776 B1**
(45) Date of publication and mention of the grant of the patent: **03.09.2008**
(21) Application number: 02022717.9
(22) Date of filing: 11.10.2002
(51) Int. Cl.: A61K 31/728, A61K 9/12, A61P 11/00, A61P 11/02, A61P 11/06

(54) **A pharmaceutical colloidal preparation comprising hyaluronic acid biopolymers useful in the treatment of respiratory diseases**
Eine pharmazeutische kolloidale Zusammensetzung enthaltend Hyaluronsäure-Biopolymere zur Behandlung von Atemwegserkrankungen
Une composition pharmaceutique colloide comprenant des biopolymères d'acide hyaluronique pour le traitement des maladies respiratoires

(43) Date of publication of application: 14.04.2004
(73) Proprietor: Petrigni, Giuseppe, 20122 Milano (IT); Allegra, Luigi, 20123 Milano (IT)
(72) Inventor: Petrigni, Giuseppe, 20122 Milano (IT); Allegra, Luigi, 20123 Milano (IT)
(74) Representative: Beneduce, Gianna

(56) References cited:
- WO-A-02/09728
- WO-A-95/26735
- WO-A-97/25051
- US-A- 4 784 990

## Description

The present invention relates to a colloidal pharmaceutical preparation useful in the treatment of diseases of the upper and lower respiratory tract, and lung parenchyma.

The pharmaceutical preparation object of the present invention contains, as an active ingredient, a colloidal mixture of hyaluronic acids of different molecular weights, in addition to a suitable diluent. The preparation has shown to be particularly effective in the treatment of diseases of the upper and lower airways, and the lung parenchyma.

Another object of the present invention is the use, in diseases of the upper and lower airways, and the lung parenchyma, of the colloidal preparation, consisting of a mixture of hyaluronic acids of different molecular weights, optionally additioned to a suitable diluent, by systemic, oral and topical administration.

The preparation object of the present invention is preferably administered locally in aerosol form, within the airways by means of nebulisers, spray, or in the form of inhalatory powder or it may be substituted and/or integrated by local instillation. The preparation is also suitable for systemic administration, by means of subcutaneous, intramuscular, intradermic injection, or oral administration in the form of tablets, or transcutaneously by means of skin patches.

For specific therapeutic indications, systemic administration may be added to topical inhalation or instillation.

A further object of the present invention is the process employed to obtain the preparation of the present invention which contains a suitable mixture of different molecular weight hyaluronic acids, as active ingredients, in addition to a suitable diluent, that has shown to be effective in the treatment of diseases of the upper and lower respiratory tract, and the lung parenchyma.

### Background of the invention

Hyaluronic acid is a naturally occurring biopolymer exerting numerous biological activities in bacteria and more complex animal organisms, including man. Naturally occurring hyaluronic acid may be found in highly evolved animal tissues, mostly present in intercellular spaces. It may be found in practically every part of living organisms, its distribution being almost ubiquitous in tissues and in organ parenchyma, reaching greater concentrations in lax connective tissue and specifically in vitreous humour and joint synovial fluid. The serum levels of hyaluronic acid are generally between 10 and 100 µg/L. The lung content of the different forms of hyaluronic acid varies between 15 and 170 µe/gr of dry weight. Lung hyaluronic acid is primarily found in perialveolar and peribronchial tissue. It is drained through the lymphatic system and finally broken down in hepatic lymph nodes. Up to a short time ago, hyaluronic acid was traditionally derived from natural deposits such as cockscomb, or bovine connective tissue by separation methods consisting of an enzyme digestion, a specific separation intended to remove the proteins and a purification to obtain the crude extract. These methods present several disadvantages connected to high production costs, poor control over molecular weight, and risk of viral infection.

Hyaluronic acid may also be produced by means of bio-fermentation. Biotechnological processes such as Gram-positive bacterial fermentation have recently been used: this technique allows the formation of potentially unlimited resources of hyaluronic acid biopolymers devoid of the afore mentioned drawbacks. A mucous capsule of hyaluronic acid envelops Streptococci (Gram-positive bacteria). Hyaluronic acid produced by streptococcal fermentation varies in molecular weight from 1 to 4 MDa.

Hyaluronic acid is a member of the glycosaminoglycan family, composed of linear, non-ramified, disaccharide polyanionic chains wherein disaccharide units, composed of n-acetylglucosamine and glucuronic acid, bonded each other by glucoside links, are repeat structures. Hyaluronic acid molecular weight may vary from hundreds of thousands to millions of Daltons. Unlike other glycosaminoglycans, hyaluronic acid contains no sulphate groups, is free from covalent bonds with proteins, and is thought to possess, amongst all mucopolysaccharide substances, the simplest chemical structure.

In body tissues, hyaluronic acid is found bound to cell membranes, joined to other macromolecules, or as a free polysaccharide. It is unique in its ability to bond and retain large amounts of water in the interfibrillar spaces - up to 6 litres of water per gram of hyaluronic acid - thus forming the backbone of the amorphous colloidal matrix acting as cement between cells and connective fibres. As a result of its influence on interstitial volume, water conductivity, and macromolecule diffusion, hyaluronic acid plays a significant effect in regulating microcirculatory exchanges.

Within the skin, solutions of hyaluronic acid in water give rise to gels that act as dampers.

Hyaluronic acid plays an important role in the body, both for its mechanical and transport properties. It has shown to be important in different tissue functions such as hydration, lubrication, solute transport, cell detachment and migration. Hyaluronic acid also plays a central role in controlling cell growth and differentiation in addition to tissue morphogenesis.

Hyaluronic acid solutions are typically viscoelastic and pseudoplastic. The viscoelastic properties of hyaluronic acid, important in its use as a biomaterial, are determined by the concentration and molecular weight of its chains. The molecular weight of the different forms of hyaluronic acid is polydispersed and highly variable, ranging between 10,000 and 10,000,000 Da.

Bronchial asthma is a disease displaying acute reversible bronchoconstriction associated with airway inflammation and hyper-responsiveness leading to excessive bronchospasm in response to a variety of external stimuli that may be specific (allergens), non specific chemicals (histamine, metacholine), or physical agents (ultrasound nebulized water, cold air, polluting gases). It is a well known fact that hyaluronio acid is currently used in many fields of medicine: it is employed in different settings such as orthopaedics and surgery. The use of hyaluronic acid in the treatment of diseases of the respiratory tract, specifically involving the lung and bronchi, is described in the International Patent Application WO 95/26735: salt, alcohol, aqueous, or dimethylsulphoxide solutions of hyaluronic acid are administered in the trachea of experimental animals at a daily dose between 10 µg/kg and 2 mg/kg.

In United States Patent No.4,784,990 is describes a method for obtaining high molecular weight hyaluronic acids by cultivating cultures of the genus Streptococcus, in particular a mutant strain of Streptococcus zooepidemicus. By said method is obtained a mixture of hyaluronic acids having a random selection of molecular weights ranging from about 1,000 kDa to about 4,000 kDa; mixtures containing hyaluronic acids having lower molecular weight, between about from 700 kDa to 1,500 kDa, with an indication of cosmetic grade and mixtures containing hyaluronic acids having an higher average molecular weight from about 2,000 kDa to about 4,000 kDa with an indication of generic clinical grade.

WO/09728 describes a method of preventing or treating a large number of diseases, among which is also cited asthma, by administering pharmaceutical compositions containing, a large selection of compounds among which, hyaluronic acids are included. These compositions may be a mixture of two or more molecular weight ranges of hyaluronic acids or salts or derivatives thereof, having molecular weight <100,000 Da combined with hyaluronic acid, or salts or derivatives thereof, having molecular weight>1,000,000 Da.

### Description of the invention

It has been found, and this is the object of the present invention, that colloidal pharmaceutical preparations containing hyaluronic acid biopolymers, having different molecular weight and suitably mixed each other in a proper ratio and dispersed in a suitable diluent, showed to be very effective in the treatment of diseases of the upper and lower airways, and the lung parenchyma.

In particular among the diseases of the airways are included: tonsillitis, pharyngitis-laringitistracheatis, nasal polyps, vocal chord polyps, sinus polyposis, acute and chronic rhinitis, allergic rhinitis, vasomotor rhinitis, otitis, diseases of the sinuses, allergic and non allergic bronchial asthma, both simple and obstructive chronic bronchitis, chronic obstructive pulmonary disease (COPD), primary and secondary pulmonary emphysema, usual interstitial pneumonitis, fibrosing alveolitis, interstitial fibrosis of both known and unknown origin, vascular and bronchial pulmonary dysplasia, bronchiectasis, pulmonary manifestations of collagen diseases, work related pulmonary diseases, both primitive (carcinoma and sarcoma) and metastatic lung tumours, neoplasms of the pleura, pneumonia (bacterial, viral, fungal, parasitic), irritant-related (gas or chemical) lung injury, primary and metastatic diseases of the pleura, ARDS and IRDS (Adult and Infant Respiratory Distress Syndrome), pulmonary tuberculosis, pulmonary embolism and infarction, cartilage diseases involving the larynx, the trachea, or the bronchi, upper and lower airway vascular diseases, neurogenic diseases of the upper and lower airways, muscular-tendon diseases of the upper and lower airways, pulmonary mycosis.

Examples of suitable diluents of the mixture of hyaluronic acids are distilled water, saline, dimethylsulfoxide, or alcohol solutions.

More specifically, hyaluronic acids or hyaluronic fractions forming the mixture of the invention have a molecular weight comprised in the range from 50 kDa and 4,000 kDa. The fractions are mixed in defined proportions on the basis of their respective molecular weights.

Among the preferred pharmaceutical preparations of the present invention are mixtures containing hyaluronic acids having molecular weight values representing multiples, in respect to the molecolar weight of the lowest molecular weight component, the different molecular weight hyaluronic acids being optionally each other in a unitary ratio. Generally, the number of hyaluronic acids of different molecular weight contained in the mixture may vary considerably and also may vary the ratio in which the different molecular weight hyaluronic acids are each other mixed in the mixture. Preferably the number of different molecular weight hyaluronic acids present in the mixture varies from 2 to 9, and more preferably from to 7.

Preferred mixtures of hyaluronic acids are those containing 100 kDa and 400 kDa hyaluronic acids and optionally their multiples: among them are particularly preferred those mixtures of hyaluronic acids having molecolar weight: 100 kDa, 200 kDa, 400 kDa, 800 kDa, 1,200 kDa and those mixtures of hyaluronic acids having molecular weight: 400 kDa, 800 kDa, 1,200 kDa, 1,600 kDa and 2,000 kDa.

### Synthesis scheme for a 10% hyaluronic acid colloidal preparation (hereafter defined as preparation A).

Solutions of hyaluronic acid with molecular weight ranging between 200 and 4.000kDa were prepared. The process of preparation is essentially based on the condensation reaction between plant-extracted glucuronic acid and a solution of hydrolysed chitine (85-90% of dry acetylglucosamine), in the presence of a biological catalyst, at a temperature of 18-20°C. A disaccharide mixture, 90% of which in dry weight, having substantially a structure identical to that of the hyaluronic acid basic monomer, and 10%, in dry weight, being the polymers, up to 4,000 kDa, of the basic monomer, is obtained. The mixture, in the form of a 10% aqueous solution, is a transparent colloidal fluid. The above mentioned proportions between monomers and polymers were chosen in order to optimize the degree of viscosity without interfering with the required activities of the preparation.

Pour 890 g of demineralized water into a steel turboemulsor, bring the temperature of the fusor to 18-20°C and create a vacuum. Start the mixer at low speed and, after having controlled the temperature (18-20°C), slowly add 100 g of the hyaluronic acid mixture and 10 of methyl parabenzoate. The mass obtained is then homogenized at low speed for approximately 5 minutes. Once the product is homogenized, the mixer is kept running for an extra 30 minutes. The resulting product is kept under vacuum for 60 minutes, and the apparatus then discharged.

With the aid of a mucorheometer device (Eslab, Milan, Italy), viscosity (h) and elasticity (G) of preparation **A** were evaluated at different temperatures. No correlation was found between temperature and elasticity, whereas an inverse correlation was demonstrated between viscosity and temperature, considering that the preparation was obtained at 18-20°C. Elongability (spinnability) values of preparation A were also determined.

| Viscosity (h): | | |
|---|---|---|
| at 5°C | h(mPas) | 7200.20 (immediate) |
| | h(mPas) | 6446.77 (after 5 minutes) |
| at 21°C | h(mPas) | 6001.79 (immediate) |
| | h(mPas) | 5931.37 (after 5 minutes) |
| at 37°C | h(mPas) | 5676.34 (immediate) |
| | h(mPas) | 5642.63 (after 5 minutes) |

| | | |
|---|---|---|
| Spinnability: at room temperature = 18 mm | | |

### Synthesis scheme for a 4.5% hyaluronic acid colloidal preparation (hereafter defined as preparation B).

The procedure is similar to the above-described one, using 950 g of demineralized water, 45 g of the hyaluronic acid mixture, and 5 g of methyl parabenzoate. The colloidal preparation B showed the following viscosity (h) values:

| | | |
|---|---|---|
| at 37°C | h(mPas) | 95.79 (immediate) |
| | h(mPas) | 77.93 (after 5 minutes). |

| | | |
|---|---|---|
| Spinnability: at room temperature = 5 mm | | |

The preparation object of the invention is preferably administered locally in aerosol form, with the aid of a nebulizer or spray, but it may be instilled locally or administered systemically.

The therapeutic dose of hyaluronic acid delivered varies according to age, patient conditions, and route of administration. The therapeutic dose for aerosol administration varies between 20 mg/kg and 0.2 mg/kg daily.

In the following Tables are given the relevant data obtained from clinical trials performed in asthmatic patients by means of aerosol administration using a pneumatic aerosol device (Nuovo Nebula, Markos, Monza, Italy or Clemny, Chiesi, Parma, Italy). Specifically have been examined:
a 10% colloidal preparation (preparation **A),** nebulizing 1 mL, diluted to 5 mL by adding saline solution, obtaining a final concentration of 2%, and
a 4.5% colloidal preparation (preparation **B),** nebulizing 1 mL, diluted to 4 mL by adding saline solution, reaching a final concentration of 1.125%.

The aerosol granulometry of the former colloidal preparation (10%) has been tested by diluting the preparation 50, 100, 150 fold in saline solution. An API aersizer apparatus (Amherst, Mass., USA) was employed.

A bimodal granulometry distribution was detected as shown by an "irregular" Gauss curve for aerosols with granules between 8 and 30 µ in diameter (presumably involved in the drug's activity along the upper airways) and a "regular" Gauss curve for granules ranging 0.8-4 µ in diameter (presumably active along the lower airways and the lung parenchyma).

Differences in the granulometric distribution were also detected using the pneumatic aerosolizer Nebula Nuovo compared to other devices, in addition to differences related to the use of different vials on the same device, and related to whether the "bell" technique was used or not. Therefore, in order to optimize hyaluronic acid therapeutic aerosol administration, studies are needed to precisely identify the anatomical target/s to be reached so as to employ the aerosol device and accessories most suitably indicated for the clinical conditions of the patient and the desired therapeutic goal.

### I) Protective efficacy towards the bronchoconstrictive effects of different non specific challenges (non allergic) in asthmatic patients.

The protective effects towards bronchoconstriction induced by specific and non specific challenges (Allegra L., Braga P.C., Dal Negro R. Methods in Asthmology, Springer, Berlin & Milan, 1996) has been evaluated by using:
(1) exercise testing by means of a 1,000 m run to be performed within 10 minutes. The test was carried out on subjects with baseline FEV₁ values ≥ 80% of predicted values.
(2) ultrasound nebulized distilled water challenge; inhalation of a nebulised hypotonic solution (distilled water) obtained by means of a 4 minute ultrasonic aerosol device. The test was carried out on subjects with baseline FEV₁ values ≥ 80% of predicted values.
(3) metacholine challenge: aerosol administration of metacholine at geometrically progressive increasing doses. The test was interrupted once the PD₂₀FEV₁ (dose of metacholine capable of reducing FEV₁ values by 20% compared to baseline) was reached, and the value was recorded in µg/mL. Each aerosol metacholine dose was administered immediately following the previous dose. The test was performed on subjects with baseline FEV₁ values ≥ 80% of predicted values.
(4) Specific bronchial provocation challenge with specific allergens: administration of Dermatophagoides pteronyssinus in patients with house dust mite-induced rhinitis or asthma.

The test was performed on subjects with baseline FEV₁ values ≥ 80% of predicted values. Exercise testing (1) was performed, on two non-consecutive days, on 19 patients (16 males). Patients were pre-treated 30 minutes before the test with placebo (9‰ saline solution) or with preparation **A** aerosol administration. The study was performed in a randomized, cross-over, single blind fashion. Among the patients, 6 males were aged between 4 and 12 years; 6 males were aged between 13 and 16 years, and the remaining 7 patients (4 males and 3 females) were aged between 20 and 36 years.

Table 1 shows variations in FEV₁ ± s.e. (litres), as this is generally considered the most significant spirometric variable. For all 19 patients, spirometry values were obtained 5 minutes following termination of exercise testing.

**TABLE 1 Exercise testing: evaluation of the efficacy of preparation A**

| | Pre-treatment | | | | | |
|---|---|---|---|---|---|---|
| | | Placebo | Hyaluronic acid (preparation **A)** | | | |
| | Baseline | | After challenge | Baseline | | After challenge |
| FEV₁ (L) | 3.03 | | 2.00 (-34%) | 2.98 | | 2.60 (-13%) |
| Mean ± s.e. | ±0.24* | | ±0.24# | ±0.25* | | ±0.22# |
| P | | < 0.01 | | | < 0.01 | |

| | | | | | | |
|---|---|---|---|---|---|---|
| *p=NS #p=<0.01 | | | | | | |

Following stratification based on age in years, the bronchoconstrictive effect of exercise testing was found to be statistically relevant in children, adolescents, and adults (p < 0.01 within each subgroup). The protective efficacy of the tested hyaluronic acid colloidal preparation, compared to placebo, was equally statistically significant in all three age groups (p < 0.02 for adolescents and adults, p < 0.01 in children).

Ultrasonically nebulized distilled water testing (2) was performed, on two non-consecutive days, on 20 patients (13 males), aged between 15 and 62 years. Patients were pre-treated 30 minutes before the test with placebo (9‰ saline solution) or with preparation **A** aerosol administration. The study was performed in a randomized, cross-over, single blind fashion.

Table 2 shows variations in FEV₁ ± s.e. (litres), obtained for all 20 patients, 3-5 minutes following termination of challenge testing.

**TABLE 2 Ultrasonically nebulized distilled water challenge: evaluation of the efficacy of preparation A.**

| | Pre-treatment | | | |
|---|---|---|---|---|
| | Placebo | | Hylaluronic acid (preparation A) | |
| | Baseline | After challenge | Baseline | After challenge |
| FEV₁(L) | 3.65 | 2.39 (-35%) | 3.68 | 2.78 (-24%) |
| Mean ±s.e. | ±0.17* | ±0.17# | ±0.18* | ±0.19# |
| **P** | | < 0.001 | < 0.01 | |

| | | | | |
|---|---|---|---|---|
| *p=NS #p=<0.05 | | | | |

Using the same ultrasonically nebulized distilled water challenge (2) on two non-consecutive days, 6 patients (5 males), aged between 18 and 46 years were examined. Patients were pre-treated 30 minutes before the test with placebo (9‰ saline solution) or with preparation B aerosol administration. The study was performed in a randomized, cross-over, single blind fashion.

Table 3 shows variations in FEV₁ ± s.e. (litres), obtained for all 6 patients, 3-5 minutes following termination of challenge testing

**TABLE 3 Ultrasonically nebulized distilled water challenge: evaluation of the efficacy of preparation B.**

| | Pre-treatment | | | |
|---|---|---|---|---|
| | Placebo | | Hyaluronic acid (preparation B) | |
| | Baseline | After challenge | Baseline | After challenge |
| FEV₁ (L) | 4.04 | 2.81 (-30%) | 4.05 | 3.38 (-16%) |
| Mcan ± s.c. | ±0.31* | ± 0.30^{#} | ±0.28* | ±0.31 |
| P | < 0.01 | | < 0.01 | |

| | | | | |
|---|---|---|---|---|
| * p = NS #p = <0.01 | | | | |

Metacholine challenge was performed, on two non-consecutive days, on 10 patients (6 males), aged between 18 and 54 years. Patients were pre-treated 30 minutes before the test with placebo (9‰ saline solution) or with preparation A aerosol administration. The study was performed in a randomized, cross-over, single blind fashion.

**TABLE 4 Metacholine challenge: evaluation of the efficacy of preparation A**

| | Pre-treatment | | |
|---|---|---|---|
| | Placebo | | Hyaluronic acid (preparation A) |
| PD₂₀ FEV₁ | 185 ± 29 | | 789 ± 290 (+332) |
| P | | <0.03 | |

The mixture of hyaluronic acids, named preparation **A,** was tested on 49 asthmatic patients (35 males, 14 females), and preparation **B** was tested on 6 asthmatic patients (5 males and 1 female). Both aerosol preparations were administered 30 minutes prior to patient exposure to non specific challenges (exercise in 19 patients: ultrasonic nebulized distilled water in 26 patients: increasing doses of aerosolized metacholine in 10 patients). Among these asthmatic patients, some had extrinsic asthma, whereas others had multifactorial forms of the disease. In all patients, both preparations **A** and **B** proved to be effective, with non detectable differences between patients with different forms of asthma. The doses employed were significantly protective in all cases, both in terms of spirometric parameters (as shown by FEV₁ values) in exercise testing and ultrasonically nebulized distilled water testing, and in terms of the dose of bronchoconstrictive substance (metacholine) required to reduce the FEV₁ values by 20% compared to baseline (PD₂₀). Preparation **A** was shown to be particularly effective during exercise and metacholine testing. Considering exercise testing, it must be added that the protection was statistically significant in all categories obtained by stratifying patients undergoing exercise testing: "children", "adolescents", and "adults". Moreover, for ultrasonically nebulized distilled water challenge, preparation **B** proved to be as protective as preparation **A.**

### II) Protective effects towards the bronchoconstrictive effects of specific challenge (allergen) in asthmatic patients.

Allergen testing was performed on 2 patients (1 male, 1 female) aged 16 and 26 years, with documented allergy to Dermatophagoides pteronyssinus (Dpt). The patients first underwent respiratory function testing, and hence specific bronchial provocation testing by nebulising 120 µg of the allergen (Dpt). Both patients were pre-treated on two non-consecutive days with placebo (9‰ NaCl solution) or hyaluronic acid (preparation **B)** aerosol 30 minutes prior to testing. Repeat FEV₁ determinations at 1; 5: ; 15; 30 minutes were performed following challenge termination. For the first patient, the point of greatest "drop" in FEV₁ (after placebo pre-treatment) was observed at + 5 minutes post-challenge, and at + 30 minutes post-challenge for the second patient. Preparation B offered complete protection for the patients, given that in both cases it was not associated with the fall in FEV₁ observed following placebo pre-treatment.

### III) Protective efficacy towards the obstructive effects on nasal airways induced bv specific challenge (allergen) in patients with rhinitis.

It was demonstrated the protective effects towards nasal obstruction induced by specific challenge in patients with allergic rhinitis towards house dust mite by means of intranasal spray administration of Dermatophagoides pteronyssinus, 60 µg per nostril. Rhinomanometry was then performed. Four patients (3 males) aged between 16 and 31 years underwent the above challenge following pre-treatment, on two non-consecutive days, 30 minutes before the test with placebo (9‰ NaCl solution) or with preparation B intranasal aerosol administration. Repeat rhinomanometry testing was performed to evaluate cumulative airflow across both nostrils at 1; 5; 15; 30; 60 minutes from termination of the challenge, in a randomized, cross-over, single blind fashion.

Table 5 shows variations in the most relevant rhinomanometry index, i.e. cumulative airflow (L.min⁻¹) + s.c. across both nostrils during the expiratory phase of a normal respiratory cycle (at a constant pressure of 75 mPs), calculated as the maximal drop in airflow during the first 60 minutes following inhalation of placebo or preparation **B.**

**TABLE 5: Intranasal inhalation of specific allergen challenge. Evaluation of the protective effects of preparation B.**

| (cumulative expiratory flow for both nostrils in L.min⁻¹) | | | | |
|---|---|---|---|---|
| | Pre-treatment | | | |
| | Placebo | | Hyaluronic acid (preparation B) | |
| | Baseline | Post-Dpt | Baseline | Post-Dpt |
| Case 1 | 599 | 63 | 987 | 626 |
| Case 2 | 427 | 189 | 225 | 247 |
| Case 3 | 525 | 254 | 403 | 470 |
| Case 4 | 185 | 25 | 932 | 484 |
| Mean ± s.e. | 434 ± 90 | 133 ± 53 | 637 ± 190 | 457 ± 78 |
| p | < 0.01 | | NS | |

## Claims

1. A pharmaceutical colloidal preparation active for the treatment of respiratory diseases of the upper and lower airways, and the lung parenchyma, **characterized by** the fact that it contains, suitably dispersed in a proper diluent, hyaluronic acid biopolymers having a plurality of different molecular weights that are multiples of the molecular weight of the lowest molecular weight hyaluronic acid present in the mixture and the overall range of molecular weights of said hyaluronic acids is from 50 kDa to 4,000 kDa.

2. A pharmaceutical colloidal preparation according to claim 1, **characterized by** the fact that the number of hyaluronic acids having different molecular weight present in the mixture varies from 2 to 9.

3. The pharmaceutical colloidal preparation according to claim 2, **characterized by** the fact that the number of hyaluronic acids having different molecular weight present in the mixture varies from 5 to 7.

4. The pharmaceutical colloidal preparation according to claim 1, **characterized by** the fact that the proper diluent is selected in the group consisting of distilled water, saline solution, dimethylsulphoxide and suitable alcohol solutions.

5. A pharmaceutical colloidal preparation, according to any of the previous claims, **characterized by** the fact that the hyaluronic acids having different molecular weights present in the mixture are each other in a unitary ratio.

6. The pharmaceutical colloidal preparation according to claim 5, **characterized by** the fact that the hyaluronic acids present in the mixture have molecular weight of 400, 800, 1,200, 1,600, and 2000 kDa respectively and that the different molecular weight hyaluronic acids present in the mixture are in the 1:1:1:1:1 ratio.

7. The pharmaceutical colloidal preparation according to claim 5, **characterized by** the fact that the hyaluronic acids present in the mixture have a molecular weight of 100, 200, 400, 800, and 1,200 kDa respectively and that the different molecular weight hyaluronic acids present in the mixture are in the 1:1:1:1:1 ratio.

## Patentansprüche

1. Kolloidale pharmazeutische Zubereitung, aktiv zur Behandlung von Atemwegserkrankungen der oberen und unteren Atemwege und des Lungen-Parenchyms, **dadurch gekennzeichnet, dass** sie entsprechend in einem geeigneten Verdünnungsmittel dispergiert Hyaluronsäurebiopolymere mit einer Vielzahl von unterschiedlichen Molekulargewichten, welche Vielfache des Molekulargewichts der in der Mischung vorliegenden Hyaluronsäure mit dem niedrigsten Molekulargewicht sind, enthält und der Gesamtbereich der Molekulargewichte der Hyaluronsäuren von 50 kDa bis 4.000 kDa reicht.

2. Kolloidale pharmazeutische Zubereitung nach Anspruch 1, **dadurch gekennzeichnet, dass** die Zahl der in der Mischung vorliegenden Hyaluronsäuren mit unterschiedlichem Molekulargewicht von 2 bis 9 variiert.

3. Kolloidale pharmazeutische Zubereitung nach Anspruch 2, **dadurch gekennzeichnet, dass** die Zahl der in der Mischung vorliegenden Hyaluronsäuren mit unterschiedlichem Molekulargewicht von 5 bis 7 variiert.

4. Kolloidale pharmazeutische Zubereitung nach Anspruch 1, **dadurch gekennzeichnet, dass** das geeignete Verdünnungsmittel ausgewählt ist aus der Gruppe, bestehend aus destilliertem Wasser, Kochsalzlösung, Dimethylsulfoxid und geeignete Alkohollösungen.

5. Kolloidale pharmazeutische Zubereitung nach einem der vorhergehenden Ansprüche, **dadurch gekennzeichnet, dass** die in der Mischung vorliegenden Hyaluronsäuren mit unterschiedlichem Molekulargewicht zueinander in einem ganzzahligen Verhältnis stehen.

6. Kolloidale pharmazeutische Zubereitung nach Anspruch 5, **dadurch gekennzeichnet, dass** die in der Mischung vorliegenden Hyaluronsäuren jeweils Molekulargewichte von 400, 800, 1.200, 1.600 und 2.000 kDa aufweisen und dass die in der Mischung vorliegenden Hyaluronsäuren mit unterschiedlichem Molekulargewicht im Verhältnis 1:1:1:1:1 stehen.

7. Kolloidale pharmazeutische Zubereitung nach Anspruch 5, **dadurch gekennzeichnet, dass** die in der Mischung vorliegenden Hyaluronsäuren jeweils Molekulargewichte von 100, 200, 400, 800, 1.200 kDa aufweisen und dass die in der Mischung vorliegenden Hyaluronsäuren mit unterschiedlichem Molekulargewicht im Verhältnis 1:1:1:1:1 stehen.

## Revendications

1. Préparation pharmaceutique colloïdale active pour le traitement des maladies respiratoires des voies aériennes supérieures et inférieures, et du parenchyme pulmonaire, **caractérisée par le fait qu'**elle contient, dispersés de façon appropriée dans un diluant adapté, des biopolymères d'acide hyaluronique ayant une pluralité de masses moléculaires différentes qui sont des multiples de la masse moléculaire de l'acide hyaluronique de plus faible masse moléculaire présent dans le mélange, la plage totale de masses moléculaires desdits acides hyaluroniques étant de 50 kDa à 4 000 kDa.

2. Préparation pharmaceutique colloïdale selon la revendication 1, **caractérisée par le fait que** le nombre d'acides hyaluroniques ayant des masses moléculaires différentes présent dans le mélange varie de 2 à 9.

3. Préparation pharmaceutique colloïdale selon la revendication 2, **caractérisée par le fait que** le nombre d'acides hyaluroniques ayant des masses moléculaires différentes présent dans le mélange varie de 5 à 7.

4. Préparation pharmaceutique colloïdale selon la revendication 1, **caractérisée par le fait que** le diluant adapté est choisi parmi le groupe constitué par de l'eau distillée, une solution saline, du diméthylsulfoxyde et des solutions d'alcool adaptées.

5. Préparation pharmaceutique colloïdale selon l'une quelconque des revendications précédentes, **caractérisée par le fait que** les acides hyaluroniques ayant des masses moléculaires différentes présents dans le mélange sont, l'un vis-à-vis de l'autre, dans un ratio unitaire.

6. Préparation pharmaceutique colloïdale selon la revendication 5, **caractérisée par le fait que** les acides hyaluroniques présents dans le mélange ont une masse moléculaire de 400, 800, 1 200, 1 600 et 2 000 kDa respectivement, et que les acides hyaluroniques de masses moléculaires différentes présents dans le mélange sont dans un ratio de 1:1:1:1:1.

7. Préparation pharmaceutique colloïdale selon la revendication 5, **caractérisée par le fait que** les acides hyaluroniques présents dans le mélange ont une masse moléculaire de 100, 200, 400, 800 et 1 200 kDa respectivement, et que les acides hyaluroniques de masses moléculaires différentes présents dans le mélange sont dans un ratio de 1:1:1:1:1.
